# FASCICULE DE BREVET EUROPEEN

(11) **EP 4 479 120 B1**
(45) Date de publication et mention de la délivrance du brevet: **11.02.2026**
(21) Numéro de dépôt: 23710074.8
(22) Date de dépôt: 15.02.2023
(51) Int. Cl.: A61M 35/00, A45D 34/00, A45D 34/04, B65D 83/00, B05B 11/00, B05B 1/30, B05B 9/08, B05B 12/00, B65D 83/766

(54) **DISTRIBUTEUR DE PRODUIT FLUIDE**
SPENDER FÜR FLÜSSIGE PRODUKTE
FLUID PRODUCT DISPENSER

(30) Priorité: 17.02.2022 FR 2201402; 04.08.2022 FR 2208075
(43) Date de publication de la demande: 25.12.2024
(73) Titulaire: APTAR France SAS, 27110 Le Neubourg (FR)
(72) Inventeur: AILLIOT, Antoine, 27190 FERRIÈRES-HAUT-CLOCHER (FR); COURDIER, Denis, 01100 OYONNAX (FR); DAVIOT, Stéphane, 27110 GRAVERON SEMERVILLE (FR); HAQUET, Timothée, 27110 VITOT (FR); MOREAU, Francis, 76300 SOTTEVILLE LES ROUEN (FR); PEREZ, Emmanuel, 27520 BOISSEY-LE-CHATEL (FR)
(74) Mandataire: CAPRI
(86) Numéro de dépôt international: PCT/FR2023/050203
(87) Numéro de publication internationale: WO 2023/156734

(56) Documents cités:
- WO-A1-2015/011427
- WO-A2-2012/131320
- GB-A- 2 127 494
- US-A- 4 090 646
- US-A1- 2018 295 968

## Description

La présente invention concerne un distributeur de produit fluide comprenant deux modules séparables, à savoir :
- un module de réservoir comprenant un étui recevant une cartouche de produit fluide définissant une paroi mobile (par exemple un piston) sollicitée par un ressort reposant sur un appui, de manière à déplacer la paroi mobile pour refouler du produit fluide hors de la cartouche de produit fluide, et
- un module de distribution obturant l'étui et comprenant un clapet de sortie commandé par un organe d'actionnement pour commander le passage du produit fluide issu sous pression de la cartouche de produit fluide.

Le module de réservoir est connecté de manière amovible au module de distribution, de manière à donner accès à l'étui et pouvoir ainsi extraire la cartouche de produit fluide de l'étui, notamment pour la remplacer par une nouvelle cartouche. Le domaine privilégié de la présente invention est celui de la cosmétique ou de la pharmacie : le distributeur permettant par exemple d'appliquer de manière continue une crème ou une pommade sur la peau, une muqueuse ou les cheveux.

Le remplacement de la cartouche dans ce type de distributeur est souvent une opération compliquée, du fait qu'il faut reconnecter les deux modules avec le ressort qui appuie sur la paroi mobile de la cartouche. Cela demande une dextérité et de la force. De plus, la cartouche est généralement obturée par un opercule qui est percé lors de l'assemblage des deux modules. Une mauvaise manipulation peut alors conduire à une perte de produit fluide.

Le but de la présente invention est de remédier aux inconvénients précités de l'art antérieur en définissant un distributeur dans lequel l'assemblage des deux modules et la mise sous pression du produit fluide sont consécutifs, et non pas simultanés : la mise sous pression étant effectuée une fois l'assemblage des modules réalisé.

Pour atteindre ce but, la présente invention propose que l'appui du ressort soit mobile par rapport à l'étui entre une position active dans laquelle le ressort est comprimé et une position de repos dans laquelle le ressort est détendu, permettant ainsi de connecter le module de réservoir au module de distribution avec l'appui en position de repos, puis ensuite de déplacer l'appui en position active. Ainsi, le ressort n'agit pas sur la cartouche lors de l'assemblage des modules : le produit fluide stocké dans la cartouche est à la pression atmosphérique. Ce n'est que dans un second temps que l'appui est ramené dans sa position active, comprimant ou armant ainsi le ressort qui va alors à nouveau agir sur la paroi mobile de la cartouche et mettre le produit fluide qu'elle contient sous pression.

Selon un mode de réalisation, le ressort peut être disposé entre une pièce de poussée et une pièce d'appui qui sont mobiles par coulissement l'une par rapport à l'autre ainsi que par rapport à l'étui, la pièce de poussée formant une tête de poussée en contact de la paroi mobile de la cartouche de produit fluide et la pièce d'appui formant l'appui, le ressort agissant entre la tête de poussée et l'appui, de manière à solliciter les pièces de poussée et d'appui en éloignement l'une de l'autre. En d'autres termes, les pièces de poussée et d'appui coulissent l'une dans l'autre en comprimant/détendant le ressort. La tête de poussée et l'appui sont avantageusement situés à des extrémités opposées des pièces de poussée et d'appui, de sorte que le ressort s'étend sur toute la longueur des deux pièces.

Selon une caractéristique de l'invention, la pièce d'appui peut être solidaire de l'étui en position active, la pièce de poussée, lors de l'ouverture du clapet de sortie, étant mobile par rapport à l'étui et à la pièce d'appui en position active. En somme, seule la pièce de poussée se déplace dans l'étui pour pousser la paroi mobile de la cartouche.

Avantageusement, la pièce d'appui et la pièce de poussée sont en butée mutuelle en position de repos avec le ressort détendu, la pièce d'appui et la pièce de poussée en butée mutuelle étant mobiles par rapport à l'étui en l'absence d'une cartouche pleine de produit fluide. Le ressort n'a pas besoin être totalement détendu : il suffit qu'il soit dans son état le plus détendu, de préférence proche de son état totalement détendu. Le ressort peut légèrement solliciter les deux pièces en butée mutuelle, ou pas. Lorsque la cartouche a été retirée de l'étui, l'ensemble formé par les pièces de poussée et d'appui en butée mutuelle peut coulisser dans l'étui, au moins sur une certaine course.

Selon une autre caractéristique de l'invention, la pièce d'appui peut être encliquetée de manière amovible sur un profil d'accrochage de l'étui en position active. Ainsi, il suffit d'enfoncer à fond la pièce d'appui dans l'étui pour réaliser l'encliquetage. Selon une caractéristique particulièrement avantageuse, la pièce de poussée comprend un profil de libération qui agit sur la pièce d'appui pour la libérer de son encliquetage avec l'étui, lorsque la cartouche de produit fluide est vidée. De préférence, la pièce d'appui forme au moins une patte souple d'encliquetage pourvue d'une dent d'encliquetage adaptée à venir en prise encliquetée avec le profil d'accrochage de l'étui, le profil de libération de la pièce de poussée déformant la patte souple d'encliquetage de manière à désengager la dent d'encliquetage du profil d'accrochage de l'étui. Avantageusement, le ressort sollicite les pièces de poussée et d'appui en éloignement l'une de l'autre, lorsque le profil de libération agit sur la pièce d'appui, de sorte que la pièce d'appui, une fois libérée de son encliquetage avec l'étui, est déplacée par le ressort hors de l'étui en position de repos, donnant ainsi à l'utilisateur une indication visuelle, sonore et/ou tactile que la cartouche de produit fluide est vide.

Selon un autre aspect de l'invention, la pièce de poussée peut être déplaçable dans l'étui entre une position de butée pleine et une position de butée vide, correspondant respectivement aux états plein et vide de la cartouche de produit fluide. Avantageusement, l'appui est en position active lorsque la pièce de poussée est déplacée par le ressort de sa position de butée pleine jusqu'à proximité de sa position de butée vide. De préférence, l'appui est déplacé en position de repos lorsque la pièce de poussée atteint sa position de butée vide.

Par ailleurs, le clapet de sortie est avantageusement sollicité dans un état fermé par le produit fluide sous pression.

Selon un autre mode de réalisation avantageux, le module de réservoir peut comprendre en outre un satellite, qui coopère à la fois avec l'étui et la pièce d'appui pour permuter entre les positions active et de repos. De préférence, le satellite est prisonnier de la pièce d'appui avec un déplacement axial limité et un déplacement en rotation, le satellite venant sélectivement en prise stable avec l'étui en position active. Par ailleurs, la pièce d'appui peut comprendre au moins une came d'entrainement en rotation pour solliciter le satellite en rotation. Avantageusement, l'étui peut comprendre au moins une came de blocage et une came d'éjection, le satellite comprenant au moins un ergot qui glisse sur les cames de blocage et d'éjection sous l'action du ressort, qui agit sur le satellite par l'intermédiaire la pièce d'appui. L'étui peut former une butée axiale, le satellite étant alors sollicité contre cette butée axiale par la pièce d'appui.

Selon un mode de réalisation pratique,
- l'étui peut comprendre au moins une came de blocage et une came d'éjection reliées par une paroi d'arrêt, une butée axiale et au moins une cheminée axiale,
- la pièce d'appui peut comprendre une butée de limitation de course axiale et au moins une came d'entrainement en rotation,
- le satellite peut comprendre au moins un ergot, qui définit une surface de glissement, une couronne de butée et des dents.

Dans ce cas, les dents viennent en prise avec la came d'entrainement en rotation pour solliciter le satellite en rotation lors des phases de compression du ressort. La couronne de butée vient en prise avec la butée de limitation de course axiale lors des phases de détente du ressort. L'ergot se déplace dans la cheminée axiale et entre les cames de blocage et d'éjection et la butée axiale sous la sollicitation de la pièce d'appui. La surface de glissement vient en contact glissant sur les cames de blocage et d'éjection. L'ergot vient en contact à la fois avec la came de blocage et la paroi d'arrêt en position active. La came d'entrainement en rotation entraine le satellite en rotation lorsque l'ergot est en contact de la butée axiale. Ainsi, le cycle complet de l'ergot est le suivant : l'ergot est d'abord déplacé axialement dans la cheminée axiale par appui sur la pièce d'appui jusqu'à venir en contact de la butée axiale : l'ergot est alors déplacé en rotation sous l'action de la came d'entrainement en rotation. L'ergot vient ensuite en contact de la came de blocage lors d'un relâchement de la pièce d'appui : l'ergot glisse sur la came de blocage jusqu'en contact de la paroi d'arrêt, marquant la position active. L'ergot glisse ensuite contre la paroi d'arrêt par appui sur pièce d'appui jusqu'à venir en contact de la butée axiale : l'ergot est alors déplacé en rotation sous l'action de la came d'entrainement en rotation. L'ergot vient alors en contact de la came d'éjection lors d'un relâchement de la pièce d'appui. L'ergot glisse enfin sur la came d'éjection jusqu'à tomber dans une autre cheminée axiale.

Ce mécanisme et ce fonctionnement est similaire à ceux des stylos à pointe rétractable par appui sur un bouton d'extrémité. Un premier appui sur le bouton suivi d'un relâchement permet de sortir la mine du stylo et de la bloquer en position active d'écriture. Un second appui suivi d'un relâchement permet de rétracter la mine. Ce même principe est mis à profit dans la présente invention pour armer/désarmer le ressort. En partant de la position active, un premier appui sur la pièce d'appui suivi d'un relâchement permet de désarmer le ressort et un second appui suivi d'un relâchement permet à nouveau d'armer le ressort.

L'invention définit également un procédé de chargement d'un distributeur tel que défini ci-dessus, comprenant les étapes successives suivantes :
a) disposer l'appui en position de repos,
b) déconnecter le module de réservoir du module de distribution,
c) introduire une cartouche de produit fluide dans le module de réservoir,
d) connecter le module de réservoir au module de distribution,
e) déplacer l'appui en position active.

Les étapes a) et b) peuvent éventuellement être inversées : l'essentiel étant que la connexion soit réalisée avec l'appui en position de repos.

Avantageusement, l'appui revient automatiquement en position de repos lorsque la cartouche de produit fluide est vidée. L'étape a) est donc automatique et n'a pas besoin de l'intervention de l'utilisateur. On a vu précédemment que la pièce de poussée, lorsqu'elle arrive en bout de course, agit sur la pièce d'appui pour la libérer de son encliquetage avec l'étui.

La présente invention repose sur le fait de désarmer le ressort pour pouvoir connecter les deux modules sans être gêné par la pression exercée par le ressort. Ce désarmement peut résulter d'une manipulation de l'utilisateur, mais de préférence, il est automatiquement déclenché lorsque la cartouche a été vidée de son contenu. Ainsi, l'utilisateur n'a pas à se soucier du désarmement du ressort et reçoit en outre une indication visuelle, sonore et/ou tactile comme quoi la cartouche est vide. En effet, la libération de l'appui peut bien entendu se voir, puisqu'il se déplace par rapport à l'étui, mais elle peut également générée un son et/ou un petit choc dans le distributeur que l'utilisateur peut ressentir dans sa main.

GB2127494 décrit un distributeur de produit fluide de l'état de la technique.

L'invention, qui est définie à la revendication 1 et plus avant aux revendications 2-18, sera maintenant plus amplement décrite en référence aux dessins joints, donnant à titre d'exemple non limitatif, un mode de réalisation de l'invention.

Sur les figures :
La figure 1 est une vue en perspective et partiellement en transparence d'un distributeur de produit fluide de l'invention dans un état de fonctionnement,
La figure 2 est une vue similaire à celle de la figure 1 avec le distributeur dans un état de repos,
La figure 3a est une vue perspective éclatée du module de distribution du distributeur des figures 1 et 2,
La figure 3b est une vue en perspective éclatée du module de réservoir du distributeur de la figure 1,
La figure 3c est une vue en perspective éclatée de la cartouche de produit fluide du distributeur des figures 1 et 2,
Les figures 4a, 4c, 4d, 4e et 4f sont des vues en coupe transversale longitudinale à travers le module de réservoir du distributeur de l'invention dans différentes conditions visant à illustrer le fonctionnement de ce module de réservoir,
La figure 4b est une vue fortement agrandie d'un détail de la figure 4a,
La figure 5 est une vue en perspective du module de distribution du distributeur de l'invention,
Les figures 6a et 6b sont des vues en coupe transversale longitudinale à travers le module de distribution de la figure 5, respectivement en position de repos et en position actionnée, et.
Les figures 7a et 7b sont des vues en section transversale à travers le distributeur selon une second mode de réalisation de l'invention, respectivement en position de repos et de fonctionnement,
La figure 8 est une vue découpée en perspective laissant apparaitre l'intérieur du distributeur,
Les figures 9, 10 et 11 sont des vues en perspective agrandie des pièces visibles sur la figure 8, et
Les figures 12a à 12j sont des schémas illustrant les différentes étapes du fonctionnement du distributeur des figures 7a à 11.

Le distributeur de produit fluide qui a été utilisé pour illustrer la présente invention est d'un type particulier, puisqu'il s'agit d'un applicateur comprenant une tête d'application D7 permettant non seulement de distribuer le produit fluide, mais également de l'appliquer sur la surface cible souhaitée, qui peut être la peau, les ongles, les cheveux, etc. Il faut comprendre que l'invention n'est pas limitée à ce type particulier de distributeur/applicateur, mais qu'elle s'applique à tout type de distributeurs.

Le distributeur de l'invention comprend deux sous-ensembles distincts que l'on peut raccorder ensemble de manière amovible, à savoir un module de réservoir R et un module de distribution D, qui peuvent être assemblés et séparés au moyen d'une connexion amovible ou réversible, telle qu'une connexion par vissage, par encliquetage, par baïonnette, etc. Dans le distributeur illustrant l'invention, la connexion amovible est une connexion par baïonnette mettant en œuvre un logement adéquat D32 pour la réception d'un ergot R13. Ainsi, par rotation relative entre le module de réservoir R et le module de distribution D, l'ergot R13 peut être inséré/extrait du logement D32. On peut prévoir plusieurs logements D32 et plusieurs ergots R13 pour une connexion équilibrée.

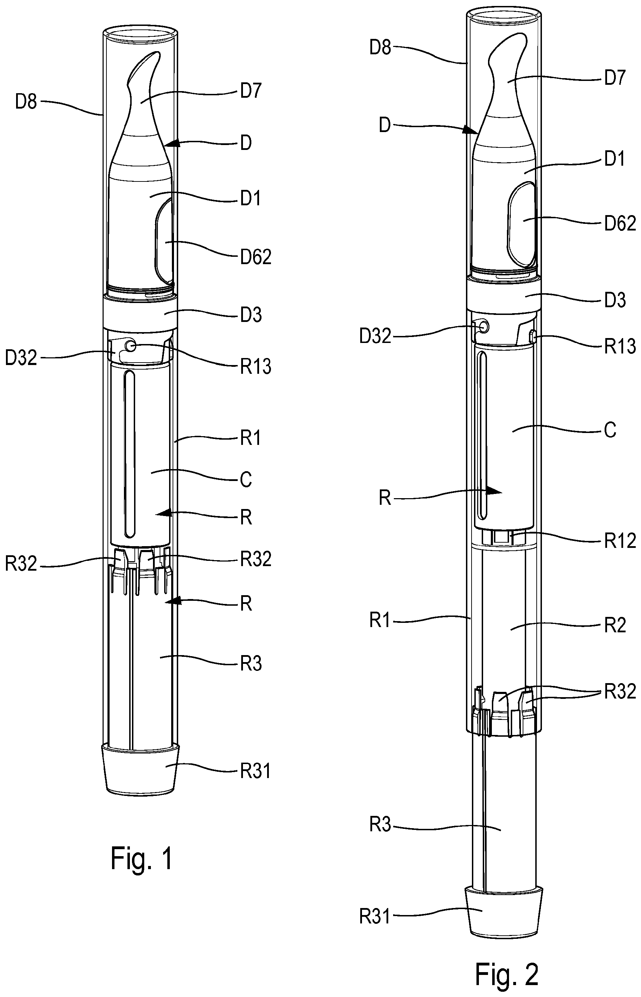

De manière générale, le module de réservoir R comprend un étui R1 destiné à recevoir de manière amovible une cartouche de produit fluide C. Pour solliciter cette cartouche C, le module de réservoir R comprend une pièce de poussée R2 qui coopère avec une pièce d'appui R3. Bien que non visible sur les figures 1 et 2, un ressort agit entre la pièce de poussée R2 et la pièce d'appui R3. On peut voir sur les figures 1 et 2 que la pièce d'appui R3 comprend un appui R31, ainsi que des pattes souples d'encliquetage R32. Le module de réservoir R sera plus amplement décrit ci-après.

Le module de distribution D, tel que visible sur les figures 1 et 2, comprend un corps D1, à l'extrémité supérieure duquel est montée la tête de distribution d'application D7. A son extrémité opposée, le module de distribution D comprend une embase D3 solidaire du corps D1 et formant le logement D32. Le corps D1 forme une fenêtre dans laquelle est disposé un poussoir D62. Optionnellement, le module de distribution D comprend un capot de protection amovible D8, qui vient en prise avec le corps D1 ou l'embase D3.

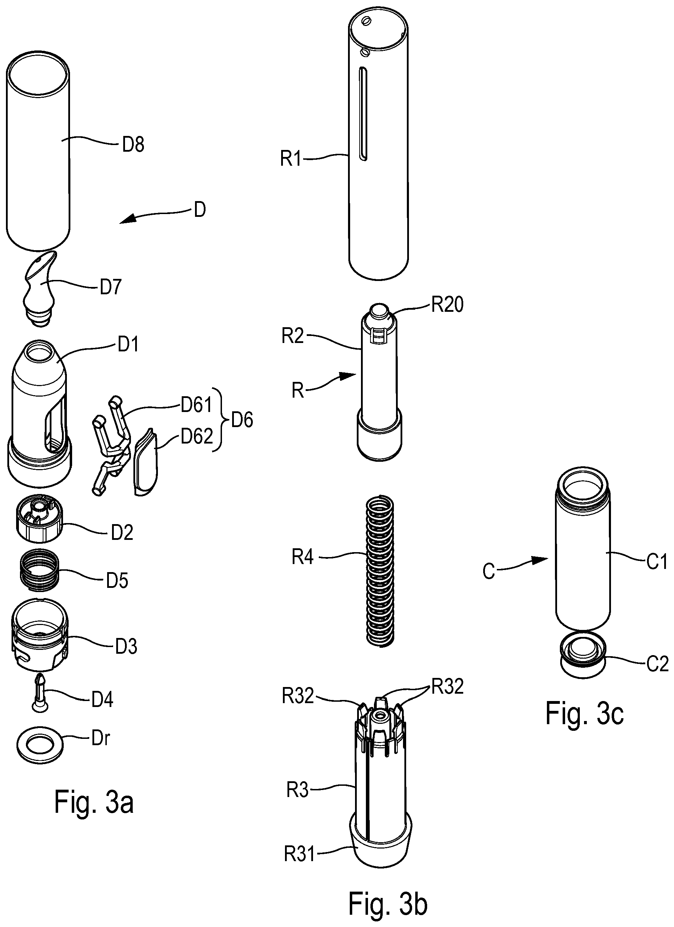

En se référant à la figure 3a, on peut voir toutes les pièces constitutives du module de distribution D. Il faut bien garder à l'esprit qu'il ne s'agit que d'un mode de réalisation non limitatif. Le module de distribution D comprend le corps D1, la tête de distribution d'application D7, l'embase D3, le capot de protection D8, ainsi qu'une navette D2 supportant un clapet de sortie D4, un ressort D5, un joint plat Dr, une genouillère D61 et un poussoir D62 formant ensemble un organe d'actionnement D6. La genouillère D61 peut être réalisée en une pièce ou en deux pièces. Elle peut même être réalisée de manière monobloc avec le poussoir D62.

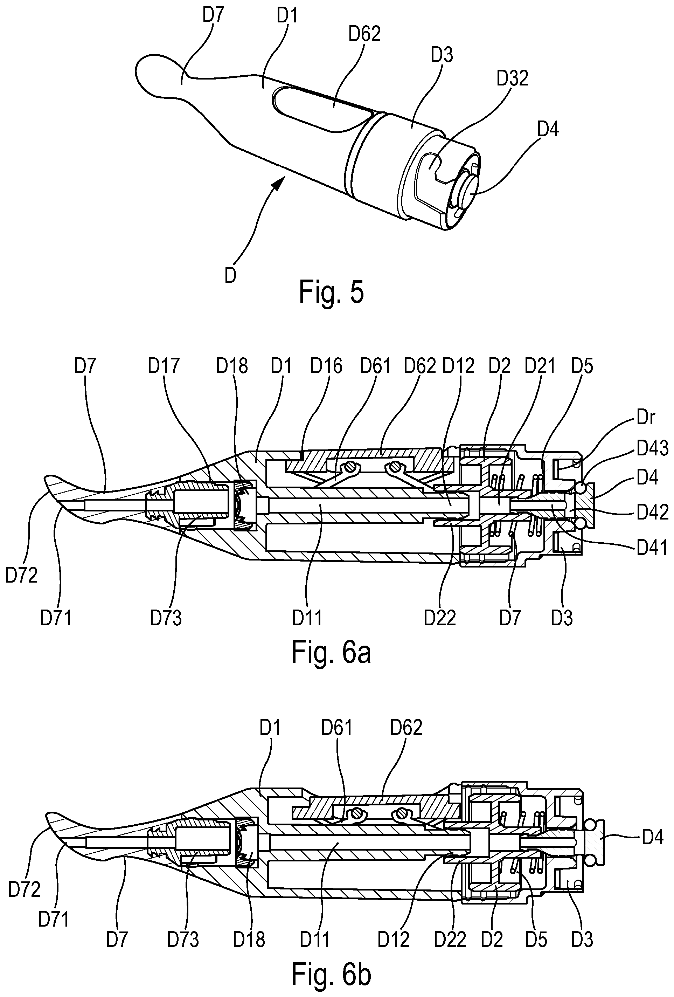

Sur la figure 5, on voit le module de distribution D en perspective à l'état monté. On peut distinguer le corps D1, la tête de distribution d'application D7, le poussoir D62, l'embase D3 avec son logement de baïonnette D32, ainsi qu'une partie du clapet de sortie D4.

Le fonctionnement de ce module de distribution D est plus compréhensible à partir des figures 6a et 6b. Le corps D1 comprend une fenêtre latérale D16, dans laquelle est logé le poussoir D62. Le corps D1 est traversé par un conduit D11 qui définit en amont un embout de coulissement D12 et comprend en aval une valve d'obturation D18. Encore plus en aval, le corps D1 comprend un logement d'ancrage D17 pour un talon d'ancrage D73 faisant partie de la tête de distribution d'application D7. On peut aussi noter que la tête D7 est traversée par un canal qui se termine par un orifice de distribution D71 au niveau d'une surface d'application D72. On peut dire que l'embout de coulissement D12 est relié de manière fluidique à l'orifice de distribution D71 en passant à travers la valve D18 et le talon d'ancrage D73.

L'embase D3 est montée fixement sur le corps D1 comme susmentionné : l'embase D3 forme un ou plusieurs logement(s) de baïonnette D32. L'embase D3 contient également la navette D2, qui comprend un manchon de coulissement D22 engagé de manière étanche et coulissante sur l'embout de coulissement D12 du corps D1. La navette D2 définit intérieurement un passage D21 qui communique avec le conduit D11. Le clapet de sortie D4 est monté fixement sur la navette D2 et définit intérieurement un canal de clapet D41, qui se prolonge par des sorties latérales D42. Le clapet de sortie D4 comprend également un élément d'étanchéité D43, qui peut se présenter sous la forme d'un joint torique qui vient en appui étanche sur l'embase D3. L'élément d'étanchéité peut également être formé sur l'embase D3. Le ressort D5 prend appui sur l'embase D3 et sollicite la navette D2 vers le corps D1, c'est-à-dire dans une direction où l'emmanchement coulissant étanche entre le manchon D22 et l'embout D12 est maximal. Ceci est représenté sur la figure 6a, qui correspond à la position de repos du module de distribution D. On peut remarquer que le joint Dr est monté dans l'embase D3.

L'organe d'actionnement D6, qui comprend la genouillère D61 et le poussoir D62, agit entre le corps D1 et la navette D2 de manière à déplacer la navette D2 en éloignement du corps D1, de manière à décoller l'élément d'étanchéité D43 de l'embase D3. Ceci est représenté sur la figure 6b. Pour ce faire, la genouillère D61 prend appui d'une part sur le corps D1 et d'autre part sur la navette D2. Par appui latéral sur le poussoir D62, la genouillère D61 est déformée, ce qui entraine le déplacement de la navette D2 vers la droite sur les figures 6a et 6b, c'est-à-dire en éloignement du corps D1. Ce déplacement s'effectue à l'encontre de la force exercée par le ressort D5. Ainsi, la genouillère D61 constitue un moyen permettant de transformer une force radiale en une force axiale agissant entre le corps D1 et la navette D2 pour commander l'ouverture du clapet de sortie D4.

Cette conception pour le module de distribution D est avantageuse, mais pas unique : d'autres conceptions sont envisageables, dans la mesure où le module de distribution D comprend un clapet de sortie commandable et peut être connecté au module de réservoir R de manière amovible.

La figure 3c montre la cartouche de produit fluide C. Celle-ci comprend un fût C1 dans lequel est engagé un piston C2, qui coulisse de manière étanche à l'intérieur du fût C1 pour faire diminuer son volume utile. A la place du piston, on peut aussi utiliser une poche souple. De manière générale, la cartouche C doit comprendre une paroi mobile, qu'elle soit coulissante comme le piston C2 ou déformable comme une poche, afin de définir un réservoir de volume variable. La cartouche C remplie de produit fluide peut être obturée par un opercule perçable ou pelable non représenté.

La figure 3b montre les éléments constitutifs du module de réservoir R, à savoir l'étui R1, la pièce de poussée R2, la pièce d'appui R3 et le ressort R4. On peut voir que module d'appui R3 comprend l'appui R31, ainsi que des pattes souples d'encliquetage R32 à l'extrémité opposée. On peut distinguer que les pattes souples R32 forment des dents d'encliquetage R321 orientées vers l'intérieur. Les pièces de poussée R2 et d'appui R3 sont destinées à être assemblées de manière à contenir le ressort R4 qui prend appui d'une part sur l'appui R31 de la pièce d'appui 3 et pousse d'autre part la tête de poussée R20 de la pièce de poussée R2 contre le piston C2 de la cartouche de produit fluide C, l'amenant ainsi à coulisser de manière étanche à l'intérieur du fût C1.

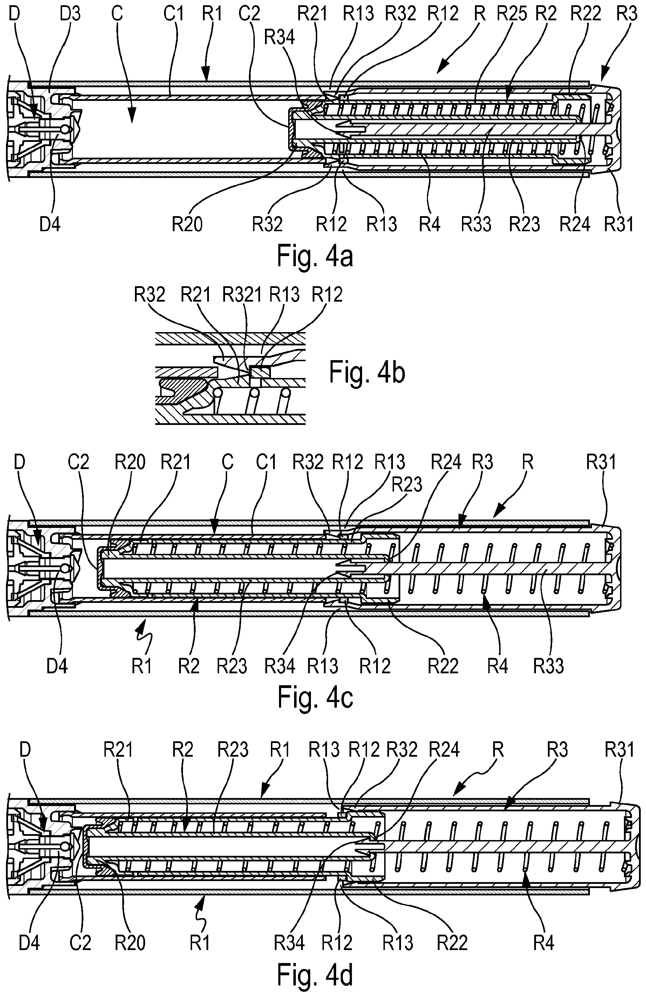

On se référera maintenant à la figure 4a qui montre le module de réservoir R dans son état de fonctionnement, correspondant à la figure 1. Sur la partie gauche de la figure 4a, on peut voir la partie inférieure du module de distribution D. L'étui R1 du module de réservoir R est obturé par le module de distribution D, de la manière susmentionnée, c'est-à-dire par une fixation amovible par baïonnette. On peut aussi remarquer que le fût C1 est en prise étanche avec l'embase D3. Ainsi, le contenu de la cartouche C communique directement avec le clapet de sortie D4. L'opercule de la cartouche C a par exemple été rompu par un profil adéquat formé par l'embase D3.

Selon l'invention, l'étui R1 forme intérieurement plusieurs profils R12, qui peuvent par exemple se présenter sous la forme de barrettes formées de manière monobloc avec l'étui R1, mais séparées de la paroi interne de l'étui R1 par un passage d'accès R13. Ceci est plus visible sur la figure 4b. Ces barrettes peuvent être rectilignes ou courbes : leurs deux extrémités se raccordant à la paroi interne de l'étui R1. Ces profils (ou barrettes) R12 sont situés sensiblement au milieu de l'étui R1 et sont répartis selon une ligne annulaire. On peut remarquer que les pattes souples d'encliquetage R32 de la pièce de poussée R3 sont engagées à travers les passages d'accès R13 de manière à ce que leurs dents d'encliquetage R321 viennent en prise encliquetée avec les profils R12. On peut aussi remarquer que la pièce de poussée R2 forme des lames élastiques R21 qui viennent en butée contre les profils R12. On peut ainsi dire que les lames élastiques R21 viennent en prise avec les profils R12 sur la même face des profils que les dents d'encliquetage R321. Dans cette position active, le ressort R4 est comprimé de manière maximale à l'intérieur de l'ensemble formé par la pièce de poussée R2b et la pièce d'appui R3. Le ressort R4 prend appui sur l'appui R31 de la pièce d'appui R3 et agit directement sur la tête de poussée R20 de la pièce de poussée R2. Le ressort R4 est engagé principalement à l'intérieurement de la pièce de poussée R2 qui forme un cylindre R25 qui se termine par un épaulement R22 de diamètre accru. La pièce de poussée R2 comprend également un tube interne R23 qui se termine par une ouverture de passage R24. La pièce d'appui R3 s'étend autour du cylindre R25 de son appui 31 jusqu'à ses pattes souples d'encliquetage R32. Intérieurement, la pièce d'appui R3 comprend une tige R33 engagée à l'intérieur du tube R23 à travers l'ouverture de passage R24. Cette tige R33 se termine par un ou plusieurs profil(s) d'accrochage R34, qui permettent l'insertion de la tige R33 dans le tube R23, mais qui empêchent son extraction par butée des profils d'accrochage R34 sur le bord de l'ouverture de passage R24, comme on le verra ci-après. D'autres moyens pour associer la tige R3 et le tube R23 sont également possibles.

Ainsi, dans cette position active, la pièce d'appui R3 est retenue dans l'étui R1 par le passage des pattes souples R32 à travers les passages d'accès R13 et leur encliquetage sur les profils (barrettes) R12 : l'appui R31 venant pratiquement en butée contre l'extrémité de l'étui R1. On peut dire que l'appui R31 est fixe par rapport à l'étui R1 dans cette position active. Le ressort R4, qui est comprimé au maximum, agit donc entre l'appui R31 et la tête de poussée R20 de la pièce de poussée R2, qui est en contact direct avec le piston C2 de la cartouche C. Le produit fluide contenu dans la cartouche C est ainsi sous pression. Il ne peut toutefois pas s'échapper, du fait que le clapet de sortie D4 est fermé.

Par appui sur le poussoir D62, le clapet de sortie D4 s'ouvre et le produit fluide sous pression dans la cartouche C est refoulé à travers le module de distribution D jusqu'à son orifice de distribution D71. La distribution de produit fluide s'accompagne d'un déplacement du piston C2 sous l'action de la tête de poussée R20, qui est sollicitée par le ressort R4 qui repose sur l'appui fixe R31 de la pièce d'appui R3. La pièce de poussée R2 se déplace à l'intérieur du fût C1, à l'exception de son épaulement R22 qui coulisse à l'intérieur de la pièce d'appui R3. Le déplacement de l'épaulement R22 dans la pièce d'appui R3 peut s'effectuer par coulissement avec ou sans contact. On peut remarquer sur la figure 4c que l'épaulement R22 s'approche des pattes souples d'encliquetage R32, qui sont en prise avec les profils R12. La tige R33 est sortie du tube R23, mais les profils d'accrochage R34 sont situés à l'intérieur du tube R23. La cartouche C est presque vide, puisque le piston C2 arrive à proximité du clapet de sortie D4.

Sur la figure 4d, la cartouche C est maintenant vide : le piston C2 étant à proximité du clapet de sortie D4. Il faut surtout remarquer que l'épaulement R22 de la pièce de poussée R2 est maintenant situé au niveau des pattes souples d'encliquetage R32, qui sont sollicitées vers l'extérieur par l'épaulement R22, de manière à se désengager de leur encliquetage avec les profils R12 de l'étui R1. L'épaulement R22 est alors en butée contre les profils R12, marquant la fin de course de la pièce de poussée R2, et de ce fait du piston C2. Sous l'effet du ressort R4, la pièce d'appui R3 a été déplacée vers la droite, conduisant à déplacer ou extraire les pattes souples d'encliquetage R32 des passages d'accès R13 et à éloigner l'appui 31 de l'extrémité de l'étui R1. Le ressort R4 est alors dans sa position de repos, détendue ou presque détendue. Cette position de repos est fixée par l'appui des profils d'accrochage R34 contre le bord de l'ouverture de passage R24.

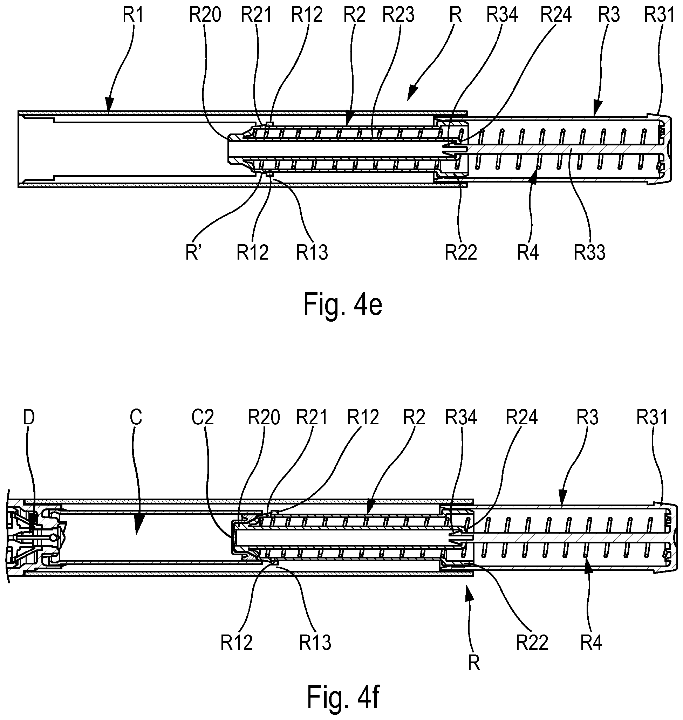

Dès lors, l'ensemble constitué par la pièce de poussée R2 et la pièce d'appui R3 est libre de coulisser à l'intérieur de l'étui R1. On peut donc déplacer cet ensemble unitaire dans la position de la figure 4e, où l'on remarque que le module de distribution D a été retiré de l'étui R1 et que la cartouche vide C a été extraite du même étui R1. Dans cette position de repos ouverte, les lames élastiques R21 ont pu se détendre hors de la cartouche C pour venir en butée contre une face des profils R12. Bien entendu, les profils d'accrochage R34 sont toujours en butée contre le bord de l'ouverture de passage R24. Le ressort R4 est détendu ou presque détendu. En tout cas, il est dans son état le plus détendu.

On comprend alors qu'il est aisé d'introduire une nouvelle cartouche remplie C à l'intérieur de l'étui R1, jusqu'à ce que son piston C2 vienne en butée contre la tête de poussée R20 de la tête de poussée R2. Le module de distribution D peut alors être remis en place sur le module de réservoir R. Avantageusement, l'embase D3 peut former un profil de perçage pour percer ou découper l'opercule qui scelle la cartouche C. On se trouve alors dans la configuration représentée sur la figure 4f. Il faut noter que la tête de poussée R20 ne sollicite pas le piston C2, étant donné que le ressort R4 est détendu. Pour armer à nouveau le distributeur, il suffit de pousser à l'aide d'un doigt sur l'appui R31 de manière à faire coulisser la pièce d'appui R3 à l'intérieur de l'étui R1 autour de la pièce de poussée R2. Le coulissement de la pièce d'appui R3 a pour effet de comprimer le ressort R4 et de solliciter la tête de poussée R20 contre le piston C2. La pièce d'appui R3 arrive dans sa position finale active, lorsque les pattes souples d'encliquetage R32 reviennent à nouveau en prise encliquetée sur les profils R12, après passage à travers les ouvertures d'accès R13. Cette position finale active est celle de la figure 4a, où l'on voit que l'appui R31 est à nouveau en butée, (ou presque) contre l'extrémité de l'étui R1.

A travers cette description complète d'un cycle de fonctionnement du distributeur, on peut voir que le déplacement de l'appui R31 par rapport à l'étui R1 permet de relâcher la pression exercée par le ressort R4, ce qui autorise l'insertion d'une nouvelle cartouche C dans l'étui R1 sans subir la poussée de la tête de poussée R20. En d'autres termes, la présente invention permet de désarmer le ressort R4 en déplaçant son appui R31. Une fois la cartouche insérée dans l'étui R1 et le module de distribution D reconnecté au module de réservoir R, le ressort R4 peut à nouveau être armé en déplaçant et bloquant l'appui R31 dans sa position de départ active.

Il faut aussi remarquer que le désarmement du ressort R4 se produit automatiquement lorsque la cartouche C est vidée de son contenu, de sorte que l'utilisateur n'a pas besoin d'agir ou de manipuler le distributeur pour désarmer le ressort R4. En effet, c'est la pièce de poussée R2 qui agit sur la pièce d'appui R3 en fin de course pour défaire la pièce de poussée R3 de sa prise avec l'étui R1. Il s'agit là d'une caractéristique particulièrement avantageuse au niveau de la gestuelle.

Les profils ou barrettes R12 de l'étui R1 remplissent plusieurs fonctions, à savoir d'arête d'encliquetage pour les pattes souples R32 et de surface de butée pour les lames élastiques R21 et l'épaulement R22.

L'épaulement R22 permettant de libérer les pattes souples d'encliquetage des profils R12 remplissent une fonction de libération et peuvent de ce fait être qualifiés de profils de libération.

La pièce de poussée R2 est prisonnière de l'étui R1, tout en coulissant entre deux butées extrêmes, à savoir une butée pleine, dans laquelle les lames élastiques R21 sont en butée contre les profils R12, correspondant à un état plein de la cartouche C et une butée vide, dans laquelle l'épaulement R22 est en butée contre les profils R12, correspondant à un état vide de la cartouche C. L'appui R3 est en position active lorsque la pièce de poussée R2 est déplacée par le ressort R4 de sa butée pleine jusqu'à proximité de sa butée vide. L'appui R31 est déplacé en position de repos lorsque la pièce de poussée R2 atteint sa butée vide.

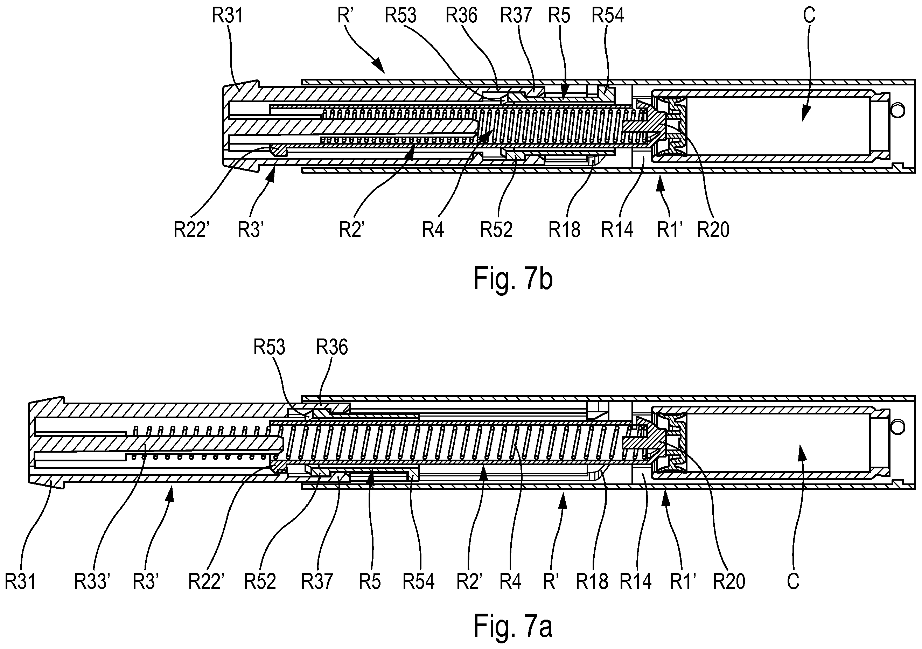

En se référant maintenant aux figures 7a, 7b, 8, 9, 10 et 11, on peut voir un second mode de réalisation, qui diffère du premier, par le mécanisme permettant d'armer et de désarmer le ressort R4. Le module de distribution D, avec son clapet de sortie D4 commandé par l'organe d'actionnement D6, peut être identique au similaire à celui du premier mode de réalisation. La cartouche C peut également être identique ou similaire.

Dans ce second mode de réalisation, le module de réservoir R se distingue par la mise en œuvre d'un satellite R5, qui agit entre la pièce d'appui R3' et l'étui R1'.

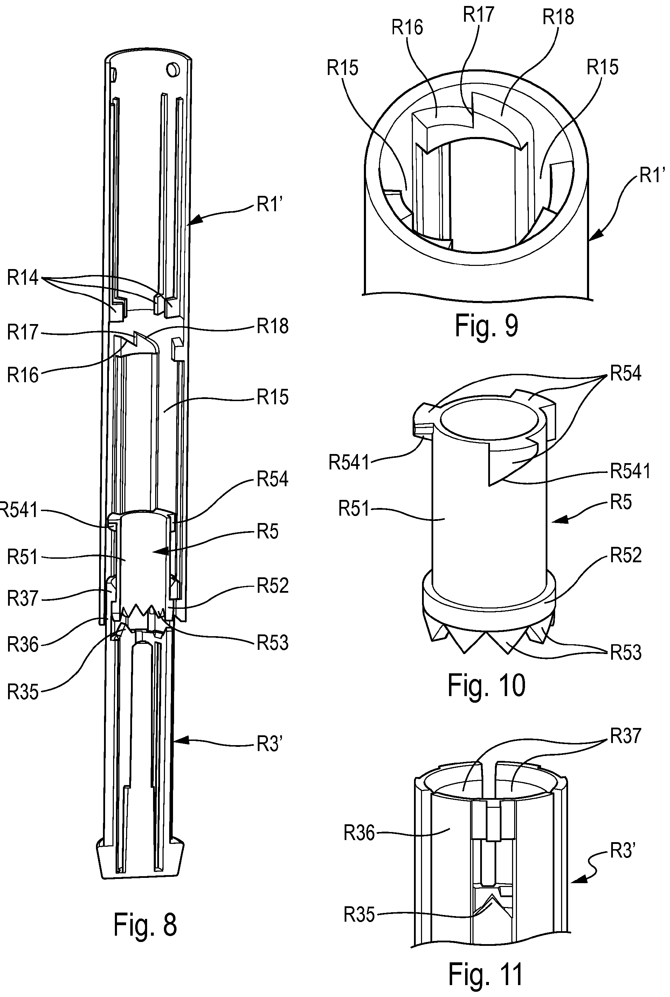

Plus précisément, l'étui R1', comparable à l'étui R1, comprend au moins une came de blocage R16 et une came d'éjection R18 reliées par une paroi d'arrêt R17. Les cames R16 et R18 sont saillie à l'intérieur de l'étui R1' et sont courbes et inclinées. La paroi d'arrêt R17 est verticale et radiale : elle s'étend dans un plan radial vertical. L'étui R1' comprend également une butée axiale R14, qui est définie par plusieurs languettes verticales radiales, qui s'étendent à l'intérieur de l'étui R1'. La butée axiale R14 est située au-dessus des cames R16 et R18, en définissant entre elles un espace de débattement. L'étui R1' définit aussi au moins une cheminée axiale R15. Comme on peut le voir sur la figure 9, la paroi interne de l'étui R1' forme trois ensembles identiques comprenant chacun une came de blocage R16, une paroi d'arrêt R17, une came d'éjection R18 et une cheminée axiale R15.

La pièce d'appui R3', comparable à l'étui R3, comprend trois cames d'entrainement en rotation R35, qui se présentent ici sous la forme de dents sensiblement triangulaires qui pointent en direction de la cartouche C. La pièce d'appui R3' comprend également trois pattes R37, qui sont légèrement déformables. Les trois cames d'entrainement en rotation R35 sont disposées entre les trois pattes R37. Les trois pattes R37 forment ensemble une butée de limitation de course axiale R38, sous la forme d'un renfort intérieur définissant un épaulement annulaire. Le ressort R4 n'est pas représenté, mais il prend appui au fond de la pièce d'appui R3', comme dans le premier mode de réalisation en R31.

Le satellite R5 est une pièce monobloc qui comprend un corps sensiblement cylindrique R51. Comme on peut le voir sur la figure 10, le satellite R5 comprend ou forme trois ergots R54 au niveau de son extrémité supérieure. Ces ergots R54 font saillie vers l'extérieur du corps R51 et définissent chacun une surface de glissement inclinée R541. A son extrémité opposée inférieure, le corps forme une couronne de butée R52 et des dents R53, qui font saillie vers l'extérieur du corps R51. Les dents R53 peuvent être adjacentes et s'étendre sur toute la périphérie du bord inférieur du corps R51. Les dents R53 sont de forme sensiblement triangulaire et pointent en direction des dents des cames d'entrainement en rotation R35.

L'étui R1', la pièce d'appui R3' et le satellite R5 coopèrent de la manière suivante. Les dents R53 du satellite R5 viennent en prise avec les dents des cames d'entrainement en rotation pour solliciter le satellite R5 en rotation lors des phases de compression du ressort R4, c'est-à-dire lorsque l'utilisateur appui sur la pièce d'appui R3' à l'encontre du ressort R4. La couronne de butée R52 vient en prise avec la butée de limitation de course axiale R37 lors des phases de détente du ressort R4, c'est-à-dire lorsque l'utilisateur relâche sa poussée sur la pièce d'appui R3'. Les ergots R54 se déplacent dans les cheminées axiales R15 et dans l'espace de débattement défini entre les cames de blocage R16 et d'éjection R18 et la butée axiale R14, sous la sollicitation de la pièce d'appui R3'. La surface de glissement R541 des ergots R54 vient en contact glissant sur les cames de blocage R16 et d'éjection R18 : les ergots venant en contact à la fois avec les cames de blocage R16 et les parois d'arrêt R17 en position active. Les dents des cames d'entrainement en rotation R35 entrainent le satellite R5 en rotation lorsque les ergots R54 sont en contact de la butée axiale R14.

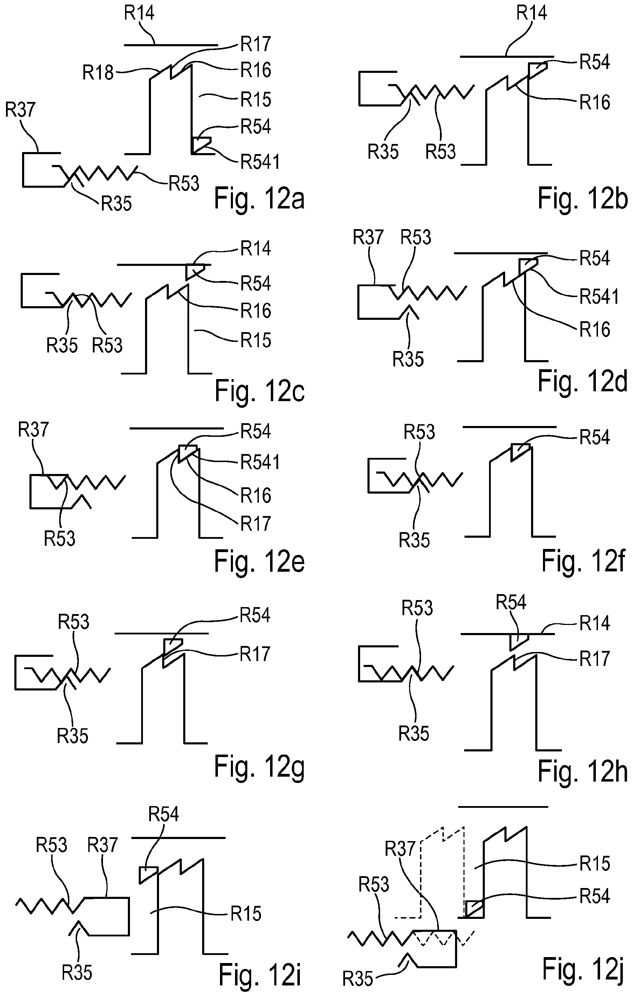

En référant aux figures 12a à 12j, un cycle complet du cheminement d'un ergot R54 est décrit.

Sur la figure 12a, l'ergot R45 est au fond de la cheminée axiale R15. Cela correspond à la position de repos désarmée de la figure 7a.

Sur la figure 12b, l'utilisateur appuie sur la pièce d'appui R3', de sorte que la dent de la came d'entrainement en rotation R35 est venue en prise entre deux dents R53 du satellite, mais ne peut s'engager à fond entre ces deux dents, en raison du guidage axial de l'ergot R54 dans la cheminée axiale R15. L'ergot R54 se déplace donc axialement dans la cheminée axiale R15, tout en étant sollicité en rotation, mais sans pouvoir tourner.

Sur la figure 12c, l'ergot R54 s'est libéré du guidage axial de la cheminée R15 et est venu en contact de la butée axiale R14 : l'ergot R54 est alors déplacé en rotation sous l'action came d'entrainement en rotation R35, dont la dent est alors engagée à fond entre les deux dents R53 du satellite. On peut remarquer que l'ergot R54 est situé partiellement au-dessus de la came de blocage R16.

Sur la figure 12d, l'utilisateur a relâché sa pression sur la pièce d'appui R3', de sorte que la dent de la came d'entrainement en rotation R35 s'est désengagée des deux dents R53 du satellite, qui sont maintenant en appui contre la butée de limitation de course axiale R38. Sous la sollicitation de la butée R38, soumise à l'action du ressort R4, la surface de glissement R541 de l'ergot R54 est descendue de manière à attraper la came de blocage R16.

Sur la figure 12e, l'ergot R54 a glissé sur la came de blocage R16 jusqu'en contact de la paroi d'arrêt R17, correspondant à la position active armée. Cette position est stable, puisque l'ergot R54 est sollicité par le ressort R4, qui agit sur la butée R38 qui est en contact des dents R53 du satellite.

Sur la figure 12f, l'utilisateur appuie sur la pièce d'appui R3', de sorte que la dent de la came d'entrainement en rotation R35 est revenue en prise entre deux dents R53 du satellite, mais ne peut s'engager à fond entre ces deux dents, en raison du guidage axial de la paroi d'arrêt R17.

Sur la figure 12g, l'ergot R54 glisse contre la paroi d'arrêt R17.

Sur la figure 12h, l'ergot R54 est libéré de la paroi d'arrêt R17 et vient en contact de la butée axiale R14. L'ergot R54 s'est déplacé en rotation sous l'action de la came d'entrainement en rotation R35 : sa dent est à nouveau engagée à fond entre les deux dents R53 du satellite.

Sur la figure 12i, l'utilisateur a relâché sa pression sur la pièce d'appui R3', de sorte que la dent de la came d'entrainement en rotation R35 s'est désengagée des deux dents R53 du satellite, qui sont maintenant en appui contre la butée de limitation de course axiale R38. Sous la sollicitation de la butée R38, soumise à l'action du ressort R4, la surface de glissement R541 de l'ergot R54 est descendue de manière à glisser sur la came d'éjection R18.

Sur la figure 12j, l'ergot R54 a quitté la came d'éjection R18 et est tombé dans une autre cheminée axiale R15. On est revenu en position de repos désarmée.

Ce type de mécanisme avec un satellite sollicité en déplacement axial et rotatif par des cames et des butées est connu en soi, mais la présente invention le met en œuvre dans une application particulière, dans laquelle il sert à armer/désarmer un ressort pour permettre un remplacement aisé de la cartouche du distributeur.

Les distributeurs de l'invention permettent de définir un procédé de chargement comprenant les étapes successives suivantes :
a) disposer l'appui en position de repos,
b) déconnecter le module de réservoir du module de distribution,
c) introduire une cartouche de produit fluide dans le module de réservoir,
d) connecter le module de réservoir au module de distribution,
e) déplacer l'appui en position active.

Grâce à l'invention, on dispose d'un module de réservoir à ressort dont le remplacement de cartouche de produit fluide est grandement facilité par l'absence de force exercée par le ressort lors de la reconnexion du module de distribution.

## Revendications

1. Distributeur de produit fluide comprenant :
a - un module de réservoir (R ; R') comprenant un étui (R1 ; R1') recevant une cartouche de produit fluide (C) définissant une paroi mobile (C2) sollicitée par un ressort (R4) reposant sur un appui (R31), de manière à déplacer la paroi mobile (C2) pour refouler du produit fluide hors de la cartouche de produit fluide (C),
b - un module de distribution (D) obturant l'étui (R1 ; R1') et comprenant un clapet de sortie (D4) commandé par un organe d'actionnement (D6) pour commander le passage du produit fluide issu sous pression de la cartouche de produit fluide (C),
dans lequel le module de réservoir (R ; R') est connecté de manière amovible au module de distribution (D), de manière à donner accès à l'étui (R1 ; R1') et pouvoir ainsi extraire la cartouche de produit fluide (C) de l'étui (R1, R1'),
dans lequel l'appui (R31) du ressort (R4) est mobile par rapport à l'étui (R1 ; R1') entre une position active dans laquelle le ressort (R4) est comprimé et une position de repos dans laquelle le ressort (R4) est détendu, permettant ainsi de connecter le module de réservoir (R ; r') au module de distribution (D) avec l'appui (R31) en position de repos, puis ensuite de déplacer l'appui (R31) en position active.

2. Distributeur selon la revendication 1, dans lequel le ressort (R4) est disposé entre une pièce de poussée (R2 ; R2') et une pièce d'appui (R3 ; R3') qui sont mobiles par coulissement l'une par rapport à l'autre ainsi que par rapport à l'étui (R1 ; R1'), la pièce de poussée (R2 ; R2') formant une tête de poussée (R20) en contact de la paroi mobile (C2) de la cartouche de produit fluide (C) et la pièce d'appui (R3 ; R3') formant l'appui (R31), le ressort (R4) agissant entre la tête de poussée (R20) et l'appui (R31), de manière à solliciter les pièces de poussée (R2 ; R2') et d'appui (R3 ; R3') en éloignement l'une de l'autre.

3. Distributeur selon la revendication 2, dans lequel la pièce d'appui (R3 ; R3') est solidaire de l'étui (R1 ; R1') en position active, la pièce de poussée (R2 ; R2'), lors de l'ouverture du clapet de sortie (D4), étant mobile par rapport à l'étui (R1 ; R1') et à la pièce d'appui (R3 ; R3') en position active.

4. Distributeur selon la revendication 2 ou 3, dans lequel la pièce d'appui (R3 ; R3') et la pièce de poussée (R2 ; R2') sont en butée mutuelle en position de repos avec le ressort (R4) détendu, la pièce d'appui (R3 ; R3') et la pièce de poussée (R2 ; R2') en butée mutuelle étant mobiles par rapport à l'étui (R1 ; R1') en l'absence d'une cartouche pleine de produit fluide (C).

5. Distributeur selon l'une quelconque des revendications 2 à 4, dans lequel la pièce d'appui (R3) est encliquetée de manière amovible sur un profil d'accrochage (R12) de l'étui (R1) en position active.

6. Distributeur selon la revendication 5, dans lequel la pièce de poussée (R2) comprend un profil de libération (R22) qui agit sur la pièce d'appui (R3) pour la libérer de son encliquetage avec l'étui (R1), lorsque la cartouche de produit fluide (C) est vidée.

7. Distributeur selon la revendication 6, dans lequel la pièce d'appui (R3) forme au moins une patte souple (R32) pourvue d'une dent d'encliquetage (R321) adaptée à venir en prise encliquetée avec le profil d'accrochage (R12) de l'étui (R1), le profil de libération (R22) de la pièce de poussée (R2) déformant la patte souple (R32) de manière à désengager la dent d'encliquetage (R321) du profil d'accrochage (R12) de l'étui (R1).

8. Distributeur selon la revendication 6 ou 7, dans lequel le ressort (R4) sollicite les pièces de poussée (R2) et d'appui (R3) en éloignement l'une de l'autre, lorsque le profil de libération (R22) agit sur la pièce d'appui (R3), de sorte que la pièce d'appui (R3), une fois libérée de son encliquetage avec l'étui (R1), est déplacée par le ressort (R4) hors de l'étui (R1) en position de repos, donnant ainsi à l'utilisateur une indication visuelle, sonore et/ou tactile que la cartouche de produit fluide (C) est vide.

9. Distributeur selon l'une quelconque des revendications 2 à 8, dans lequel la pièce de poussée (R2) est déplaçable dans l'étui (R1) entre une butée pleine et une butée vide, correspondant respectivement aux états plein et vide de la cartouche de produit fluide (C).

10. Distributeur selon la revendication 9, dans lequel l'appui (R3) est en position active lorsque la pièce de poussée (R2) est déplacée par le ressort (R4) de sa butée pleine jusqu'à proximité de sa butée vide.

11. Distributeur selon la revendication 10, dans lequel l'appui (R31) est déplacé en position de repos lorsque la pièce de poussée (R2) atteint sa butée vide.

12. Distributeur selon l'une quelconque des revendications 2 à 4, dans lequel le module de réservoir (R') comprend en outre un satellite (R5) qui coopère à la fois avec l'étui (R1') et la pièce d'appui (R3') pour permuter entre les positions active et de repos.

13. Distributeur selon la revendication 12, dans lequel le satellite (R5) est prisonnier de la pièce d'appui (R3') avec un déplacement axial limité et un déplacement en rotation, le satellite (R5) venant sélectivement en prise stable avec l'étui (R1') en position active.

14. Distributeur selon la revendication 12 ou 13, dans lequel la pièce d'appui (R3') comprend au moins une came d'entrainement en rotation (R35) pour solliciter le satellite en rotation.

15. Distributeur selon la revendication 12, 13 ou 14, dans lequel l'étui (R1') comprend au moins une came de blocage (R16) et une came d'éjection (R18), le satellite (R5) comprenant au moins un ergot (R54) qui glisse sur les cames de blocage (R16) et d'éjection (R18) sous l'action du ressort (R4) qui agit sur le satellite (R5) par l'intermédiaire la pièce d'appui (R3').

16. Distributeur selon l'une quelconque des revendications 12 à 15, dans lequel l'étui (R1') forme une butée axiale (R14), le satellite (R5) étant sollicité contre cette butée axiale (R14) par la pièce d'appui (R3').

17. Distributeur selon l'une quelconque des revendications 12 à 16, dans lequel :
- l'étui (R1') comprend au moins une came de blocage (R16) et une came d'éjection (R18) reliées par une paroi d'arrêt (R17), une butée axiale (R14) et au moins une cheminée axiale (R15),
- la pièce d'appui (R3') comprend une butée de limitation de course axiale (R38) et au moins une came d'entrainement en rotation (R35),
- le satellite (R5) comprend au moins un ergot (R54) qui définit une surface de glissement (R541), une couronne de butée (R52) et des dents (R53),
les dents (R53) venant en prise avec la came d'entrainement en rotation (R35) pour solliciter le satellite (R5) en rotation lors des phases de compression du ressort (R4), la couronne de butée (R52) venant en prise avec la butée de limitation de course axiale (R38) lors des phases de détente du ressort (R4), l'ergot (R54) se déplaçant dans la cheminée axiale (R15) et entre les cames de blocage (R16) et d'éjection (R18) et la butée axiale (R14) sous la sollicitation de la pièce d'appui (R3'), la surface de glissement (R541) venant en contact glissant sur les cames de blocage (R16) et d'éjection (R18), l'ergot venant en contact à la fois avec la came de blocage (R16) et la paroi d'arrêt (R17) en position active, la came d'entrainement en rotation (R35) entrainant le satellite (R5) en rotation lorsque l'ergot (R54) est en contact de la butée axiale (R14), de sorte que l'ergot (R54) est déplacé axialement dans la cheminée axiale (R15) par appui sur pièce d'appui (R3') jusqu'à venir en contact de la butée axiale (R14), l'ergot (R54) est alors déplacé en rotation sous l'action came d'entrainement en rotation (R35), l'ergot (R54) venant en contact de la came de blocage (R16) lors d'un relâchement de la pièce d'appui (R3'), l'ergot (R54) glissant sur la came de blocage (R16) jusqu'en contact de la paroi d'arrêt (R17), marquant la position active, l'ergot (R54) glissant contre la paroi d'arrêt (R17) par appui sur pièce d'appui (R3') jusqu'à venir en contact de la butée axiale (R14), l'ergot (R54) est alors déplacé en rotation sous l'action de la came d'entrainement en rotation (R35), l'ergot (R54) venant en contact de la came d'éjection (R18) lors d'un relâchement de la pièce d'appui (R3'), l'ergot (R54) glissant sur la came d'éjection (R18) jusqu'à tomber dans une autre cheminée axiale (R15).

18. Procédé de chargement d'un distributeur selon l'une quelconque des revendications précédentes, comprenant les étapes successives suivantes :
a) disposer l'appui (R31) en position de repos,
b) déconnecter le module de réservoir (R) du module de distribution (D),
c) introduire une cartouche de produit fluide (C) dans le module de réservoir (R),
d) connecter le module de réservoir (R) au module de distribution (D),
e) déplacer l'appui (R31) en position active.

## Patentansprüche

1. Spender für ein Fluidprodukt mit:
a - ein Reservoirmodul (R; R'), das ein Gehäuse (R1; R1') aufweist, das eine Fluidproduktkartusche (C) aufnimmt, die eine bewegliche Wand (C2) definiert, die durch eine Feder (R4) beaufschlagt ist, die sich an einem Auflager (R31) abstützt, derart, dass die bewegliche Wand (C2) bewegt wird, um Fluidprodukt aus der Fluidproduktkartusche (C) auszubringen,
b - ein Verteilmodul (D), das das Gehäuse (R1; R1') verschließt und ein Auslassventil (D4) aufweist, das durch ein Betätigungselement (D6) gesteuert ist, um den Durchtritt des unter Druck stehenden Fluidprodukts aus der Fluidproduktkartusche (C) zu steuern,
wobei das Reservoirmodul (R; R') lösbar mit dem Verteilmodul (D) verbunden ist, um Zugang zu dem Gehäuse (R1; R1') zu ermöglichen und so die Fluidproduktkartusche (C) aus dem Gehäuse (R1; R1') entnehmen zu können,
wobei das Auflager (R31) der Feder (R4) relativ zu dem Gehäuse (R1; R1') zwischen einer aktiven Position, in der die Feder (R4) komprimiert ist, und einer Ruheposition, in der die Feder (R4) entspannt ist, beweglich ist, wodurch ermöglicht wird, das Reservoirmodul (R; R') mit dem Verteilmodul (D) bei in Ruheposition befindlichem Auflager (R31) zu verbinden und anschließend das Auflager (R31) in die aktive Position zu bewegen.

2. Spender nach Anspruch 1, wobei die Feder (R4) zwischen einem Schubteil (R2; R2') und einem Auflagerteil (R3; R3') angeordnet ist, die durch Gleiten relativ zueinander sowie relativ zu dem Gehäuse (R1; R1') beweglich sind, wobei das Schubteil (R2; R2') einen Schubkopf (R20) bildet, der mit der beweglichen Wand (C2) der Fluidproduktkartusche (C) in Kontakt steht, und wobei das Auflagerteil (R3; R3') das Auflager (R31) bildet, wobei die Feder (R4) zwischen dem Schubkopf (R20) und dem Auflager (R31) wirkt, um das Schubteil (R2; R2') und das Auflagerteil (R3; R3') voneinander weg zu beaufschlagen.

3. Spender nach Anspruch 2, wobei das Auflagerteil (R3; R3') in der aktiven Position mit dem Gehäuse (R1; R1') verbunden ist, wobei das Schubteil (R2; R2') beim Öffnen des Auslassventils (D4) relativ zu dem Gehäuse (R1; R1') und zu dem Auflagerteil (R3; R3') in der aktiven Position beweglich ist.

4. Spender nach Anspruch 2 oder 3, wobei das Auflagerteil (R3; R3') und das Schubteil (R2; R2') in der Ruheposition mit entspannter Feder (R4) gegenseitig aneinander anliegen, wobei das gegenseitig aneinander anliegende Auflagerteil (R3; R3') und das Schubteil (R2; R2') relativ zu dem Gehäuse (R1; R1') beweglich sind, wenn keine mit Fluidprodukt gefüllte Kartusche (C) vorhanden ist.

5. Spender nach einem der Ansprüche 2 bis 4, wobei das Auflagerteil (R3) in der aktiven Position lösbar an einem Rastprofil (R12) des Gehäuses (R1) eingerastet ist.

6. Spender nach Anspruch 5, wobei das Schubteil (R2) ein Freigabeprofil (R22) aufweist, das auf das Auflagerteil (R3) einwirkt, um dieses aus seiner Rastverbindung mit dem Gehäuse (R1) zu lösen, wenn die Fluidproduktkartusche (C) entleert ist.

7. Spender nach Anspruch 6, wobei das Auflagerteil (R3) mindestens eine elastische Lasche (R32) bildet, die mit einer Rastzahnstruktur (R321) versehen ist, die dazu eingerichtet ist, in rastendem Eingriff mit dem Rastprofil (R12) des Gehäuses (R1) zu stehen, wobei das Freigabeprofil (R22) des Schubteils (R2) die elastische Lasche (R32) verformt, um die Rastzahnstruktur (R321) aus dem Eingriff mit dem Rastprofil (R12) des Gehäuses (R1) zu bringen.

8. Spender nach Anspruch 6 oder 7, wobei die Feder (R4) das Schubteil (R2) und das Auflagerteil (R3) voneinander weg beaufschlagt, wenn das Freigabeprofil (R22) auf das Auflagerteil (R3) einwirkt, sodass das Auflagerteil (R3), nachdem es aus seiner Rastverbindung mit dem Gehäuse (R1) gelöst ist, durch die Feder (R4) aus dem Gehäuse (R1) in die Ruheposition bewegt wird und dem Benutzer dadurch eine visuelle, akustische und/oder taktile Anzeige dafür gibt, dass die Fluidproduktkartusche (C) leer ist.

9. Spender nach einem der Ansprüche 2 bis 8, wobei das Schubteil (R2) in dem Gehäuse (R1) zwischen einem Vollanschlag und einem Leeranschlag beweglich ist, die jeweils den vollen und den leeren Zustand der Fluidproduktkartusche (C) kennzeichnen.

10. Spender nach Anspruch 9, wobei das Auflager (R3) in der aktiven Position ist, wenn das Schubteil (R2) durch die Feder (R4) von seinem Vollanschlag bis in die Nähe seines Leeranschlags bewegt wird.

11. Spender nach Anspruch 10, wobei das Auflager (R31) in die Ruheposition bewegt wird, wenn das Schubteil (R2) seinen Leeranschlag erreicht.

12. Spender nach einem der Ansprüche 2 bis 4, wobei das Reservoirmodul (R') des Weiteren einen Satelliten (R5) aufweist, der sowohl mit dem Gehäuse (R1') als auch mit dem Auflagerteil (R3') zusammenwirkt, um zwischen der aktiven Position und der Ruheposition zu wechseln.

13. Spender nach Anspruch 12, wobei der Satellit (R5) mit begrenzter axialer Verschiebung und mit Drehbeweglichkeit in dem Auflagerteil (R3') gehalten ist, wobei der Satellit (R5) selektiv in stabilem Eingriff mit dem Gehäuse (R1') in der aktiven Position steht.

14. Spender nach Anspruch 12 oder 13, wobei das Auflagerteil (R3') mindestens eine Drehmitnahmekurve (R35) aufweist, um den Satelliten (R5) in Drehung zu beaufschlagen.

15. Spender nach Anspruch 12, 13 oder 14, wobei das Gehäuse (R1') mindestens eine Blockierkurve (R16) und eine Auswurfkurve (R18) aufweist, wobei der Satellit (R5) mindestens einen Zapfen (R54) aufweist, der unter der Wirkung der Feder (R4), die über das Auflagerteil (R3') auf den Satelliten (R5) einwirkt, auf den Blockier- und Auswurfkurven (R16, R18) gleitet.

16. Spender nach einem der Ansprüche 12 bis 15, wobei das Gehäuse (R1') einen axialen Anschlag (R14) bildet, wobei der Satellit (R5) durch das Auflagerteil (R3') gegen diesen axialen Anschlag (R14) beaufschlagt ist.

17. Spender nach einem der Ansprüche 12 bis 16, wobei:
- das Gehäuse (R1') mindestens eine Blockierkurve (R16) und eine Auswurfkurve (R18) aufweist, die durch eine Anschlagwand (R17) verbunden sind, sowie einen axialen Anschlag (R14) und mindestens einen axialen Schacht (R15),
- das Auflagerteil (R3') einen axialen Hubbegrenzungsanschlag (R38) und mindestens eine Drehmitnahmekurve (R35) aufweist,
- der Satellit (R5) mindestens einen Zapfen (R54) aufweist, der eine Gleitfläche (R541) definiert, einen Anschlagkranz (R52) und Zähne (R53),
wobei die Zähne (R53) mit der Drehmitnahmekurve (R35) in Eingriff stehen, um den Satelliten (R5) während der Kompressionsphasen der Feder (R4) in Drehung zu beaufschlagen, wobei der Anschlagkranz (R52) während der Entspannungsphasen der Feder (R4) mit dem axialen Hubbegrenzungsanschlag (R38) in Eingriff kommt, wobei sich der Zapfen (R54) unter Beaufschlagung durch das Auflagerteil (R3') in dem axialen Schacht (R15) und zwischen den Blockier- und Auswurfkurven (R16, R18) sowie dem axialen Anschlag (R14) bewegt, wobei die Gleitfläche (R541) in gleitendem Kontakt mit den Blockier- und Auswurfkurven (R16, R18) steht, wobei der Zapfen (R54) in der aktiven Position sowohl mit der Blockierkurve (R16) als auch mit der Anschlagwand (R17) in Kontakt steht, wobei die Drehmitnahmekurve (R35) den Satelliten (R5) in Drehung versetzt, wenn der Zapfen (R54) mit dem axialen Anschlag (R14) in Kontakt steht, sodass der Zapfen (R54) durch Abstützung an dem Auflagerteil (R3') axial in dem axialen Schacht (R15) bis in Kontakt mit dem axialen Anschlag (R14) bewegt wird, der Zapfen (R54) anschließend durch die Drehmitnahmekurve (R35) in Drehung bewegt wird, der Zapfen (R54) beim Loslassen des Auflagerteils (R3') mit der Blockierkurve (R16) in Kontakt kommt, der Zapfen (R54) auf der Blockierkurve (R16) gleitet, bis er mit der Anschlagwand (R17) in Kontakt kommt und damit die aktive Position markiert, der Zapfen (R54) durch Abstützung an dem Auflagerteil (R3') an der Anschlagwand (R17) entlang gleitet, bis er mit dem axialen Anschlag (R14) in Kontakt kommt, der Zapfen (R54) anschließend durch die Drehmitnahmekurve (R35) in Drehung bewegt wird, der Zapfen (R54) beim Loslassen des Auflagerteils (R3') mit der Auswurfkurve (R18) in Kontakt kommt und der Zapfen (R54) auf der Auswurfkurve (R18) gleitet, bis er in einen anderen axialen Schacht (R15) fällt.

18. Verfahren zum Laden eines Spenders nach einem der vorhergehenden Ansprüche, das folgende aufeinander folgende Schritte aufweist:
a) Anordnen des Auflagers (R31) in der Ruheposition,
b) Trennen des Reservoirmoduls (R) vom Verteilmodul (D),
c) Einführen einer Fluidproduktkartusche (C) in das Reservoirmodul (R),
d) Verbinden des Reservoirmoduls (R) mit dem Verteilmodul (D),
e) Bewegen des Auflagers (R31) in die aktive Position.

## Claims

1. Fluid product dispenser comprising:
a- a reservoir module (R; R') comprising a case (R1; R1') accommodating a fluid product cartridge (C) defining a movable wall (C2) biased by a spring (R4) resting on a bearing (R31), so as to move the movable wall (C2) to push fluid product out of the fluid product cartridge (C),
b- a dispensing module (D) closing the case (R1; R1') and comprising an outlet valve (D4) controlled by an actuation member (D6) for controlling the passage of the fluid product released under pressure from the fluid product cartridge (C),
wherein the reservoir module (R; R') is removably connected to the dispensing module (D), so as to give access to the case (R1; R1') and thus be able to extract the fluid product cartridge (C) from the case (R1, R1'),
wherein the bearing (R31) of the spring (R4) is movable relative to the case (R1; R1') between an active position in which the spring (R4) is compressed and a rest position in which the spring (R4) is relaxed, thus making it possible to connect the reservoir module (R; r') to the dispensing module (D) with the bearing (R31) in the rest position, then to move the bearing (R31) in the active position.

2. Dispenser according to claim 1, wherein the spring (R4) is disposed between a thrust part (R2; R2') and a bearing part (R3; R3') which are slidably movable relative to one another as well as relative to the case (R1; R1'), the thrust part (R2; R2') forming a thrust head (R20) in contact with the movable wall (C2) of the fluid product cartridge (C) and the bearing part (R3; R3') forming the bearing (R31), the spring (R4) acting between the thrust head (R20) and the bearing (R31), so as to bias the thrust parts (R2; R2') and the bearing parts (R3; R3') away from one another.

3. Dispenser according to claim 2, wherein the bearing part (R3; R3') is secured to the case (R1; R1') in the active position, the thrust part (R2; R2'), when the outlet valve (D4) is opened, being movable relative to the case (R1; R1') and to the bearing part (R3; R3') in the active position.

4. Dispenser according to claim 2 or 3, wherein the bearing part (R3; R3') and the thrust part (R2; R2') are in mutual abutment in the rest position with the relaxed spring (R4), the bearing part (R3; R3') and the thrust part (R2; R2') in mutual abutment being movable relative to the case (R1; R1') in the absence of a full fluid product cartridge (C).

5. Dispenser according to any one of claims 2 to 4, wherein the bearing part (R3) is removably snap-fitted to a hooking profile (R12) of the case (R1) in the active position.

6. Dispenser according to claim 5, wherein the thrust part (R2) comprises a release profile (R22) which acts on the bearing part (R3) to release it from snap-fitting with the case (R1), when the fluid product cartridge (C) is emptied.

7. Dispenser according to claim 6, wherein the bearing part (R3) forms at least one flexible tab (R32) provided with a snap-fitting tooth (R321) adapted to snap-fittingly engage with the hooking profile (R12) of the case (R1), the release profile (R22) of the thrust part (R2) deforming the flexible tab (R32) so as to disengage the snap-fitting tooth (R321) of the hooking profile (R12) from the case (R1).

8. Dispenser according to claim 6 or 7, wherein the spring (R4) biases the thrust (R2) and bearing (R3) parts away from one other, when the release profile (R22) acts on the bearing part (R3), such that the bearing part (R3), once released from its snap-fitting with the case (R1), is moved by the spring (R4) out of the case (R1) into the rest position, thus giving the user a visual, audible and/or tactile indication that the fluid product cartridge (C) is empty.

9. Dispenser according to any one of claims 2 to 8, wherein the thrust part (R2) is movable in the case (R1) between a full abutment and an empty abutment, corresponding respectively to the full and empty states of the fluid product cartridge (C).

10. Dispenser according to claim 9, wherein the bearing (R3) is in the active position when the thrust part (R2) is moved by the spring (R4) from its full abutment to the proximity of its empty abutment.

11. Dispenser according to claim 10, wherein the bearing (R31) is moved into the rest position when the thrust part (R2) reaches its empty abutment.

12. Dispenser according to any one of claims 2 to 4, wherein the reservoir module (R') further comprises a planet gear (R5) which engages with both the case (R1') and the bearing part (R3') to switch between the active and rest positions.

13. Dispenser according to claim 12, wherein the planet gear (R5) is trapped in the bearing part (R3') with a limited axial movement and a rotary movement, the planet gear (R5) being selectively stably engaged with the case (R1') in the active position.

14. Dispenser according to claim 12 or 13, wherein the bearing part (R3') comprises at least one rotary drive cam (R35) for rotatably biasing the planet gear.

15. Dispenser according to claim 12, 13 or 14, wherein the case (R1') comprises at least one locking cam (R16) and one ejection cam (R18), the planet gear (R5) comprising at least one lug (R54) which slides over the locking (R16) and ejection (R18) cams under the action of the spring (R4) which acts on the planet gear (R5) through the bearing part (R3').

16. Dispenser according to any one of claims 12 to 15, wherein the case (R1') forms an axial abutment (R14), the planet gear (R5) being biased against this axial abutment (R14) by the bearing part (R3').

17. Dispenser according to any one of claims 12 to 16, wherein:
- the case (R1') comprises at least one locking cam (R16) and one ejection cam (R18) connected by a stop wall (R17), an axial abutment (R14) and at least one axial funnel (R15),
- the bearing part (R3') comprises an axial stroke-limiting abutment (R38) and at least one rotary drive cam (R35),
- the planet gear (R5) comprises at least one lug (R54) which defines a sliding surface (R541), an abutment ring (R52) and teeth (R53),
the teeth (R53) engaging with the rotary drive cam (R35) to rotatably bias the planet gear (R5) during the compression phases of the spring (R4), the abutment ring (R52) engaging with the axial stroke-limiting abutment (R38) during the relaxation phases of the spring (R4), the lug (R54) moving in the axial funnel (R15) and between the locking (R16) and ejection (R18) cams and the axial abutment (R14) under the biasing of the bearing part (R3'), the sliding surface (R541) coming into sliding contact on the locking (R16) and ejection (R18) cams, the lug coming into contact both with the locking cam (R16) and the stop wall (R17) in the active position, the rotary drive cam (R35) rotating the planet gear (R5) when the lug (R54) is in contact with the axial abutment (R14), such that the lug (R54) is moved axially in the axial funnel (R15) by pressing on the bearing part (R3') until coming into contact with the axial abutment (R14), the lug (R54) is thus rotatably moved under the action of the rotary drive cam (R35), the lug (R54) coming into contact with the locking cam (R16) when the bearing part (R3') is released, the lug (R54) sliding over the locking cam (R16) until coming into contact with the stop wall (R17), marking the active position, the lug (R54) sliding against the stop wall (R17) by pressing on the bearing part (R3') until coming into contact with the axial abutment (R14), the lug (R54) is thus rotatably moved under the action of the rotary drive cam (R35), the lug (R54) coming into contact with the ejection cam (R18) when the bearing part (R3') is released, the lug (R54) sliding over the ejection cam (R18) until falling into another axial funnel (R15).

18. Method for loading a dispenser according to any one of the preceding claims, comprising the following successive steps:
a) disposing the bearing (R31) in the rest position,
b) disconnecting the reservoir module (R) from the dispensing module (D),
c) introducing a fluid product cartridge (C) into the reservoir module (R),
d) connecting the reservoir module (R) to the dispensing module (D),
e) moving the bearing (R31) into the active position.
